# EUROPÄISCHE PATENTSCHRIFT

(11) **EP 1 488 827 B1**
(45) Veröffentlichungstag und Bekanntmachung des Hinweises auf die Patenterteilung: **14.02.2007**
(21) Anmeldenummer: 04013545.1
(22) Anmeldetag: 09.06.2004
(51) Int. Cl.: A61N 1/05

(54) **Verfahren zur Herstellung von Elektrodenstrukturen sowie Elektrodenstruktur und deren Verwendung**
Process for fabricating electrode structures and electrode structure and use thereof
Procédé de fabrication de structures d'électrodes ainsi qu'une structure d'électrode et son utilisation

(30) Priorität: 17.06.2003 DE 10327500
(43) Veröffentlichungstag der Anmeldung: 22.12.2004
(73) Patentinhaber: W.C. Heraeus GmbH, 63450 Hanau (DE)
(72) Erfinder: Frericks, Matthias, 63456 Hanau (DE); Krüger, Frank, Dr., 63486 Bruchköbel (DE)
(74) Vertreter: Kühn, Hans-Christian

(56) Entgegenhaltungen:
- EP-A- 0 711 576
- WO-A-02/052665
- WO-A-02/089907
- DE-A- 19 705 988
- US-A- 4 603 060
- US-B1- 6 266 568

## Beschreibung

Die Erfindung betrifft ein Verfahren zur Herstellung von Elektrodenstrukturen, die eine Zuleitung und eine daran anschließende Kontaktfläche aufweisen. Des weiteren betrifft die Erfindung entsprechende Elektrodenstrukturen und deren Verwendung.

Derartige Elektrodenstrukturen sind beispielsweise aus WO 02/089907 A1 bekannt. Bei den hier beschriebenen Verfahren wird eine Folie auf einen Träger aufgebracht und die Folie dann entsprechend strukturiert. Die aus den Folien hergestellten Elektroden werden zur Stimulierung als Cochlearimplantate eingesetzt. Derartige Implantate sind in WO 02/089907 A1 ausführlich beschrieben.

Aus US 6,266,568 B1 ist die Herstellung von Cochlearelektroden entnehmbar, die an einem flexiblen rohrförmigen Träger angeordnet sind.

Der vorliegenden Erfindung liegt die Aufgabe zugrunde, ein Verfahren zur Herstellung von Elektrodenstrukturen zu schaffen, die einstückig und mit geringer Baugröße preiswert hergestellt werden können. Des weiteren soll die Verwendung für die erfindungsgemäßen Elektrodenstrukturen angegeben werden.

Die Aufgabe wird durch die Merkmale der unanhängigen Ansprüche gelöst. Vorteilhafte Ausgestaltungen ergeben sich aus den Merkmalen der abhängigen Ansprüche.
Bei dem erfindungsgemäßen Verfahren wird ein flächenhaftes Elektrodenmaterial auf ein flächenhaftes Trägermaterial walzplattiert und die Dicke von Elektrodenmaterial und Trägermaterial durch Walzen reduziert, wobei das Elektrodenmaterial anschließend unter Bildung der Kontaktflächen und Zuleitungen in seiner Fläche strukturiert und definierte Teile des Elektrodenmaterials entfernt werden, wobei anschließend das am Trägermaterial befindliche Elektrodenmaterial mit einer Vergussmasse oder einer Folie überzogen und danach das Trägermaterial entfernt werden kann. Durch die dabei erfolgende Kaltverformung ist das Einstellen der mechanischen Eigenschaften in weiten Bereichen möglich. Je höher der Grad der Kaltverformung ist, um so höher ist die erreichbare mechanische Festigkeit. Das Walzplattieren ist wirtschaftlicher als die Herstellung nach dem Stand der Technik, der erforderliche Umformgrad ist frei einstellbar. So kann man relativ große Schichtstärken erreichen, so dass längere Zuleitungen mit niedrigerem elektrischen Widerstand aufgrund eines größeren Querschnitts möglich werden. Da von relativ großen Materialstärken sowohl des Elektrodenmaterials als auch des Trägermaterials beim Walzplattieren ausgegangen wird, entstehen eine Reihe von Möglichkeiten der Materialauswahl. Zweckmäßigerweise erfolgt das die Dicke reduzierende Walzen zunächst gleichzeitig mit dem Walzplattieren. Danach kann die gewünschte Enddicke über mehrere Walzstiche eingestellt werden.

Als Elektrodenmaterialien eignen sich insbesondere Metalle aus der Gruppe Pt, Ir, Au, W, Ta, Nb oder eine Legierung mit mindestens einem dieser Metalle. Zweckmäßigerweise können als Trägermaterialien Metalle oder Legierungen, vorzugsweise Cu und/oder Fe oder deren Legierungen oder ein Kunststoff verwendet werden. Die Auswahl der Materialien ermöglicht die Einstellung der Eigenschaften der Elektrodenstrukturen, etwa um weiche, leicht biegsame Elektrodenstrukturen zu erhalten oder solche mit hoher Zugfestigkeit bzw. Wechselbiegefestigkeit (bei starker mechanischer Beanspruchung oder Wechselbiegebelastung). Durch Zulegieren von Iridium zu Platin beispielsweise kann die Zugfestigkeit von unter 250 N/mm² (PtIr5, geglüht) bis über 2.000 N/mm² (PtIr30 mit hoher Kaltverformung) verändert werden. Hohe Festigkeiten sind auch erreichbar mit PtAu-Legierungen (beispielsweise PtAu5). PtW-Legierungen (etwa PtW8) haben besonders gute Wechselbiegefestigkeiten. Bei Tantal-Legierungen lässt sich mit steigendem Wolfram-Anteil die Festigkeit erhöhen. Platin und seine Legierungen weisen eine hohe Biokompatibilität auf, unabhängig von der elektrischen Polarität der Elektroden.

Die biokompatiblen Elektrodenstrukturen sind in der Regel chemisch beständig, so dass das Trägermaterial so gewählt werden kann, dass es beispielsweise durch Ätzverfahren leicht entfernt werden kann, nachdem die Elektrodenstrukturen hergestellt worden sind.

Zweckmäßigerweise werden die Strukturen fotolithografisch erzeugt, wobei die nicht die Elektrodenstruktur bildenden Teile des Elektrodenmaterials als vordefinierte Teile durch anschließendes Ätzen (insbesondere chemisches Ätzen, elektrochemisches Ätzen oder Trockenätzen) entfernt werden.

Zum fotolithografischen Strukturieren wird vorzugsweise ein fotoresistives Material in Form einer Folie oder einer Flüssigkeit verwendet. Das fotolithografische Verfahren weist eine weit höhere Flexibilität bei den herzustellenden Geometrien auf als beispielsweise die aus WO 02/089907 A1 bekannten EDM-Verfahren. Fotolithografische Verfahren sind auch wesentlich effizienter, da große Elektrodenstrukturen mit einer großen Vielzahl von Elektroden gleichzeitig behandelt werden können.

Vorzugsweise wird für das Elektrodenmaterial ein Material der Gruppe Platin-Legierungen, Gold, Gold-Legierungen, Tantal, Tantal-Legierungen, Niob, Niob-Legierungen, Kobald-Chrom-Nickel-Legierungen, Edelstahl, Nickel-Titan-Legierungen verwendet, wobei die Platin-Legierungen insbesondere mit mindestens einem Metall der Gruppe Gold, Wolfram, Iridium gebildet wird.

Die erfindungsgemäße Elektrodenstruktur aus einer Mehrzahl von gegeneinander elektrisch isolierten Elektroden, die Zuleitungen und daran anschließende Kontaktflächen aufweisen, weist Zuleitungen und zugehörige Kontaktflächen aus jeweils einem Stück auf, die aus einem gewalzten Material der Gruppe Platin-Legierungen, Gold, Gold-Legierungen, Tantal, Tantal-Legierungen, Niob, Niob-Legierungen, Kobald-Chrom-Nickel-Legierungen, Edelstahl, Nickel-Titan-Legierungen gebildet sind, wobei die Platin-Legierung insbesondere Gold, Wolfram und/oder Iridium aufweist. Die Niob-Legierung insbesondere mit Zirkonium gebildet.

Die Zuleitungen sind vorteilhafterweise zumindest teilweise in einer gemeinsamen elektrisch nicht leitfähigen Matrix gehalten, wobei die Matrix zweckmäßigerweise aus einem flexiblen Material gebildet sein kann.

Zweckmäßig ist es, dass die Elektroden planar ausgebildet sind. Des weiteren ist es zweckmäßig, dass die Elektroden eine Dicke von größer 3 µm bis etwa 15 µm aufweisen oder, dass sie, falls sehr dünne Elektroden benötigt werden, eine Dicke von etwa 0,1 µm bis 3 µm aufweisen.

Die Zuleitungen weisen zweckmäßigerweise eine Breite von größer 20 µm bis etwa 60 µm auf, können jedoch auch insbesondere eine Breite von etwa 2 µm bis 20 µm aufweisen, falls feinere Strukturen erforderlich sind.

Insbesondere ist es zweckmäßig, dass die Breite der Kontaktflächen größer oder gleich der Breite der Zuleitungen ist.

Erfindungsgemäß können die Elektrodenstrukturen als medizinisches Implantat, als Cochlearimplantat, Retinaimplantat oder als Kortikalelektrode verwendet werden.

Nachfolgend wird ein Ausführungsbeispiel der Erfindung anhand von Zeichnungen erläutert.
In den Zeichnungen zeigt:
- Fig. 1: die Herstellung der Elektrodenstrukturen und
- Fig. 2: eine fertige Elektrodenstruktur.

Zunächst wird als Elektrodenmaterial 1 PtIr10 auf das Trägermaterial 2 aus Kupfer aufgewalzt. Ausgehend von einem 1 mm dicken Blech der Platinlegierung und einem 9 mm dicken Kupferblech wird beim Walzplattieren bzw. Walzen eine Enddicke von 10 µm der Platinlegierung und 90 µm des Kupferbleches erreicht. Auf das Elektrodenmaterial 1 wird ein handelsüblicher negativer Photoresist 3 mit einer Dicke von 38 µm aufgebracht. Danach wird eine Photomaske 4 (Glasmaske) aufgebracht, die die zu realisierenden Strukturen wiedergibt. Das Material wird dazu in ein Belichtungsgerät gebracht, dessen Bestandteil die Photomaske 4 ist. Mittels UV-Licht 5 findet eine Belichtung statt. Danach wird die Struktur des Photoresists 3 entwickelt und anschließend die Schicht des Elektrodenmaterials 1 plasmageätzt.

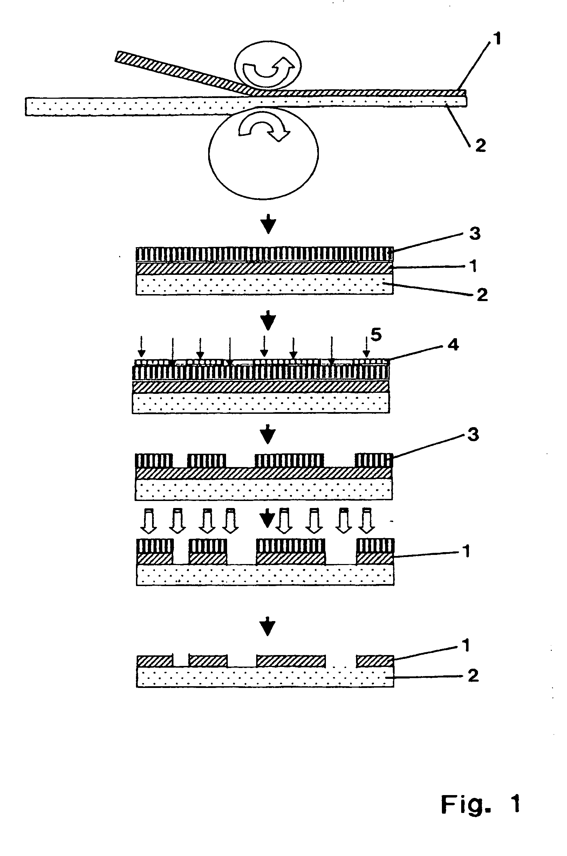

Nach Entfernen des Photoresists 3 steht die Elektrodenstruktur des Elektrodenmaterials 1 auf dem Trägermaterial 2 zur Verfügung. Der entsprechende Ablauf ist in der Figur 1 von oben nach unten dargestellt. Nachdem der Photoresist 3 entfernt wurde, werden die Elektrodenstrukturen in Silikon eingegossen und das Trägermaterial 2 wird entfernt. Neben Silikon konnen auch andere Polymere als Vergußmassen verwendet werden, wie beispielsweise Polyimid, Polyurethan, Parylene oder Polyaryletheretherketon (PEEK).

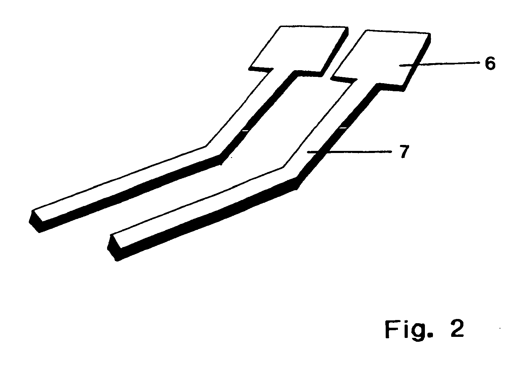

Zur Verdeutlichung der endgültigen Elektrodenstrukturen zeigt Figur 2 eine beispielhafte Ausgestaltung. Selbstverständlich können die Kontaktflächen 6 bzw. Zuleitungen 7 auch in anderer Form gestaltet sein. Beispielsweise können die Kontaktflächen 6 kreisförmig oder oval ausgebildet sein. In der Figur 2 sind die Zuleitungen leicht abgewinkelt. Dies kann erforderlich sein zur weiteren Verarbeitung bzw. in der Anwendung. Figur 2 zeigt lediglich beispielhaft zwei Kontaktflächen 6 mit Zuleitungen 7. In der konkreten Anwendung werden in der Regel mehrere derartiger Strukturen benötigt, um an vielen Stellen stimulieren zu können. Die Polymerstruktur ist in den Figuren 1 und 2 der Übersicht halber nicht dargestellt, eine derartige Anordnung ergibt sich jedoch für den Fachmann bereits aus dem Stand der Technik.

## Patentansprüche

1. Verfahren zur Herstellung von Elektrodenstrukturen, die eine Zuleitung und eine daran anschließende Kontaktfläche aufweisen, wobei ein flächenhaftes Elektrodenmaterial auf ein flächenhaftes Trägermaterial walzplattiert und die Dicke von Elektrodenmaterial und Trägermaterial durch Walzen reduziert wird, wobei das Elektrodenmaterial anschließend unter Bildung der Kontaktflächen und Zuleitungen in seiner Fläche strukturiert und definierte Teile des Elektrodenmaterials entfernt werden, wobei anschließend das am Trägermaterial befindliche Elektrodenmaterial mit einer Vergußmasse oder einer Folie überzogen und danach das Trägermaterial entfernt wird.

2. Verfahren nach Anspruch 1, **dadurch gekennzeichnet, dass** das Walzplattieren und das die Dicke reduzierende Walzen gleichzeitig erfolgen.

3. Verfahren nach Anspruch 1 oder 2, **dadurch gekennzeichnet, dass** als Elektrodenmaterial ein Metall aus der Gruppe Pt, Ir, Au, W, Ta, Nb oder eine Legierung mit mindestens einem dieser Metalle verwendet wird.

4. Verfahren nach einem der Ansprüche 1 bis 3, **dadurch gekennzeichnet, dass** als Trägermaterial ein Metall oder eine Legierung, vorzugsweise Cu und/oder Fe oder deren Legierungen oder ein Kunststoff verwendet wird.

5. Verfahren nach einem der Ansprüche 1 bis 4, **dadurch gekennzeichnet, dass** das Strukturieren fotolithografisch erfolgt und die vordefinierten Teile des Elektrodenmaterials durch anschließendes Ätzen entfernt werden.

6. Verfahren nach Anspruch 5, **dadurch gekennzeichnet, dass** zum fotolithografischen Strukturieren ein fotoresistives Material in Form einer Folie oder einer Flüssigkeit verwendet wird.

7. Verfahren nach Anspruch 5, **dadurch gekennzeichnet, dass** das Ätzen als chemisches oder elektrochemisches Ätzen oder als Trockenätzen erfolgt.

8. Elektrodenstruktur aus einer Mehrzahl von gegeneinander elektrisch isolierten Elektroden, die Zuleitungen und daran anschließende Kontaktflächen aufweisen, wobei Zuleitungen und zugehörige Kontaktflächen jeweils aus einem Stück und aus einem gewalzten Material der Gruppe Platin-Legierungen, Gold, Gold-Legierungen, Tantal, Tantal-Legierungen, Niob, Niob-Legierungen, Kobald-Chrom-Nickel-Legierungen, Edelstahl, Nickel-Titan-Legierungen gebildet sind.

9. Elektrodenstruktur nach Anspruch 8, **dadurch gekennzeichnet, dass** die Platin-Legierung insbesondere mit mindestens einem Metall der Gruppe Gold, Wolfram, Iridium gebildet ist.

10. Elektrodenstruktur nach Anspruch 8, **dadurch gekennzeichnet, dass** die Niob-Legierung insbesondere mit Zirkonium gebildet ist.

11. Elektrodenstruktur nach einem der Ansprüche 8 bis 10, **dadurch gekennzeichnet, dass** die Zuleitungen zumindest teilweise in einer gemeinsamen elektrisch nicht leitfähigen Matrix gehalten sind.

12. Elektrodenstruktur nach Anspruch 11, **dadurch gekennzeichnet, dass** die Matrix aus einem flexiblen Material gebildet ist.

13. Elektrodenstruktur nach einem der Ansprüche 8 bis 12, **dadurch gekennzeichnet, dass** die Elektroden planar ausgebildet sind.

14. Elektrodenstruktur nach einem der Ansprüche 8 bis 13, **dadurch gekennzeichnet, dass** die Elektroden eine Dicke von größer 3 µm bis etwa 15 µm aufweisen.

15. Elektrodenstruktur nach einem der Ansprüche 8 bis 13, **dadurch gekennzeichnet, dass** die Elektroden eine Dicke von etwa 0,1 µm bis 3 µm aufweisen.

16. Elektrodenstruktur nach einem der Ansprüche 8 bis 15, **dadurch gekennzeichnet, dass** die Zuleitungen eine Breite von größer 20 µm bis etwa 60 µm aufweisen.

17. Elektrodenstruktur nach einem der Ansprüche 8 bis 15, **dadurch gekennzeichnet, dass** die Zuleitungen eine Breite von etwa 2 µm bis 20 µm aufweisen.

18. Elektrodenstruktur nach einem der Ansprüche 8 bis 17, **dadurch gekennzeichnet, dass** die Breite der Kontaktflächen grösser oder gleich der Breite der Zuleitungen ist.

19. Verwendung der Elektrodenstruktur nach einem der Ansprüche 8 bis 18 zur Herstellung eines medizinischens Implantats.

20. Verwendung der Elektrodenstruktur nach einem der Ansprüche 8 bis 18 zur Herstellung eines Cochlearimplantats, Retinaimplantats oder einer Kortikalelektrode.

## Claims

1. Process for the production of electrode structures which have a supply line and a contact surface connecting thereto, a sheet-like electrode material being roll-bonded onto a sheet-like support material and the thickness of electrode material and support material being reduced by rolling, electrode material then being structured with formation of the contact surfaces and supply lines in its surface and defined parts of the electrode material being removed, the electrode material present on the support material then being covered with a potting compound or a film, and the support material then being removed.

2. Process according to Claim 1, **characterized in that** the roll-bonding and the rolling for reducing the thickness are effected simultaneously.

3. Process according to Claim 1 or 2, **characterized in that** the electrode material used is a metal from the group consisting of Pt, Ir, Au, W, Ta, Nb or an alloy with at least one of these metals.

4. Process according to any of Claims 1 to 3, **characterized in that** the support material used is a metal or an alloy, preferably Cu and/or Fe or alloys thereof, or a plastic.

5. Process according to any of Claims 1 to 4, **characterized in that** the structuring is effected photolithographically and the predefined parts of the electrode material are removed by subsequent etching.

6. Process according to Claim 5, **characterized in that** a photoresistive material in the form of a film or a liquid is used for the photolithographic structuring.

7. Process according to Claim 5, **characterized in that** the etching is effected as chemical or electrochemical etching or as dry etching.

8. Electrode structure comprising a plurality of electrodes which are electrically insulated from one another and have supply lines and contact surfaces connecting thereto, supply lines and associated contact surfaces being formed in each case from one piece and from a rolled material of the group consisting of platinum alloys, gold, gold alloys, tantalum, tantalum alloys, niobium, niobium alloys, cobalt-chromium-nickel alloys, stainless steel and nickel-titanium alloys.

9. Electrode structure according to Claim 8, **characterized in that** the platinum alloy is formed in particular with at least one metal of the group consisting of gold, tungsten and iridium.

10. Electrode structure according to Claim 8, **characterized in that** the niobium alloy is formed in particular with zirconium.

11. Electrode structure according to any of Claims 8 to 10, **characterized in that** the supply lines are held at least partly in a common electrically nonconductive matrix.

12. Electrode structure according to Claim 11, **characterized in that** the matrix is formed from a flexible material.

13. Electrode structure according to any of Claims 8 to 12, **characterized in that** the electrodes are planar.

14. Electrode structure according to any of Claims 8 to 13, **characterized in that** the electrodes have a thickness of greater than 3 µm to about 15 µm.

15. Electrode structure according to any of Claims 8 to 13, **characterized in that** the electrodes have a thickness of about 0.1 µm to about 3 µm.

16. Electrode structure according to any of Claims 8 to 15, **characterized in that** the supply lines have a width of greater than 20 µm to about 60 µm.

17. Electrode structure according to any of Claims 8 to 15, **characterized in that** the supply lines have a width of about 2 µm to 20 µm.

18. Electrode structure according to any of Claims 8 to 17, **characterized in that** the width of the contact surfaces is greater than or equal to the width of the supply lines.

19. Use of the electrode structure according to any of Claims 8 to 18 for the production of a medical implant.

20. Use of the electrode structure according to any of Claims 8 to 18 for the production of a cochlear implant, retina implant or cortical electrode.

## Revendications

1. Procédé pour la préparation de structures d'électrodes qui présentent un raccordement électrique et une surface de contact qui y est raccordée, un matériau d'électrode gauche étant fixé par laminage sur un matériau de support gauche et l'épaisseur du matériau d'électrode et du matériau de support étant réduite par laminage, le matériau d'électrode étant ensuite structuré dans sa surface avec la formation des surfaces de contact et des raccordements électriques et des parties définies du matériau d'électrode étant éliminées, le matériau d'électrode se trouvant sur le matériau de support étant ensuite recouvert avec une masse coulée ou une feuille et le matériau de support étant après cela éliminé.

2. Procédé selon la revendication 1, **caractérisé en ce que** la fixation par laminage et le laminage réduisant l'épaisseur sont réalisés simultanément.

3. Procédé selon la revendication 1 ou 2, **caractérisé en ce que** l'on utilise comme matériau d'électrode un métal du groupe Pt, Ir, Au, W, Ta, Nb ou un alliage avec au moins un de ces métaux.

4. Procédé selon l'une quelconque des revendications 1 à 3, **caractérisé en ce que** l'on utilise comme matériau de support un métal ou un alliage, de préférence Cu et/ou Fe ou des alliages de ceux-ci ou une matière plastique.

5. Procédé selon l'une quelconque des revendications 1 à 4, **caractérisé en ce que** la structure est réalisée de manière photolithographique et les parties prédéfinies du matériau d'électrode sont éliminées par décapage subséquent.

6. Procédé selon la revendication 5, **caractérisé en ce que** l'on utilise pour la structure photolithographique un matériau de photorésist dans la forme d'une feuille ou d'un liquide.

7. Procédé selon la revendication 5, **caractérisé en ce que** le décapage est réalisé comme un décapage chimique ou électrochimique ou comme un décapage à sec.

8. Structure d'électrodes constituée de plusieurs électrodes isolées électriquement les unes des autres, lesquelles présentent des raccordements électriques et des surfaces de contact qui y sont raccordées, des raccordements électriques et des surfaces de contact associées étant à chaque fois formés à partir d'un élément et d'un matériau laminé choisi parmi des alliages de platine, l'or, des alliages d'or, le tantale, des alliages de tantale, le niobium, des alliages de niobium, des alliages de cobalt-chrome-nickel, l'acier inoxydable, des alliages de nickel-titane.

9. Structure d'électrodes selon la revendication 8, **caractérisée en ce que** l'alliage de platine est en particulier formé avec au moins un métal choisi parmi l'or, le tungstène, l'iridium.

10. Structure d'électrodes selon la revendication 8, **caractérisée en ce que** l'alliage de niobium est en particulier formé avec du zirconium.

11. Structure d'électrodes selon l'une quelconque des revendications 8 à 10, **caractérisé en ce que** les raccordements électriques sont maintenus au moins partiellement dans une matrice commune non électroconductrice.

12. Structure d'électrodes selon la revendication 11, **caractérisée en ce que** la matrice est formée à partir d'un matériau flexible.

13. Structure d'électrodes selon l'une quelconque des revendications 8 à 12, **caractérisée en ce que** les électrodes sont formées de manière plane.

14. Structure d'électrodes selon l'une quelconque des revendications 8 à 13, **caractérisée en ce que** les électrodes présentent une épaisseur de plus de 3 µm à environ 15 µm.

15. Structure d'électrodes selon l'une quelconque des revendications 8 à 13, **caractérisée en ce que** les électrodes présentent une épaisseur d'environ 0,1 µm à 3 µm.

16. Structure d'électrodes selon l'une quelconque des revendications 8 à 15, **caractérisée en ce que** les raccordements électriques présentent une largeur de plus de 20 µm à environ 60 µm.

17. Structure d'électrodes selon l'une quelconque des revendications 8 à 15, **caractérisée en ce que** les raccordements électriques présentent une largeur d'environ 2 µm à 20 µm.

18. Structure d'électrodes selon l'une quelconque des revendications 8 à 17, **caractérisée en ce que** la largeur des surfaces de contact est supérieure ou égale à la largeur des raccordements électriques.

19. Utilisation de la structure d'électrodes selon l'une quelconque des revendications 8 à 18 pour la préparation d'un implant médical.

20. Utilisation de la structure d'électrodes selon l'une quelconque des revendications 8 à 18 pour la préparation d'un implant cochléaire, d'un implant de rétine ou d'une électrode corticale.
